# DEMANDE DE BREVET EUROPEEN

(11) **EP 2 727 945 A1**
(43) Date de publication de la demande: **07.05.2014**
(21) Numéro de dépôt: 13197995.7
(22) Date de dépôt: 16.06.2011
(51) Int. Cl.: C08B 37/00, C08L 5/08, A01N 25/12, A01N 43/16, A01N 63/02, A01N 63/04, A23L 2/42, A23L 2/44, A23L 2/52, A23L 3/34, C12H 1/04, A23L 1/056, A23L 1/30, C12G 1/02, C12H 1/14, A23L 3/3526, A23L 3/3544, B32B 5/16, C08B 37/08

(54) **Utilisation de chitosane sous forme de poudre comme agent antifongique dans le traitement de liquides alimentaires d'origine végétale préparé par fermentation**

(30) Priorité: 18.06.2010 FR 1054887
(62) Demande divisionnaire de: 11734166.9
(71) Demandeur: Kitozyme S.A., 4040 Herstal (BE)
(72) Inventeur: Bornet, Aurélie, 74380 LUCINGES (FR); Gautier, Sandrine, 4000 LIEGE (BE); Maquet, Véronique, 4257 BERLOZ (BE); Teissedre, Pierre-Louis, 34090 MONTPELLIER (FR); Granes, Daniels, 11000 NARBONNE (FR); Pic-Blateyron, Lucile, 34770 GIGEAN (FR)
(74) Mandataire: Blot, Philippe Robert Emile

(57) **Abrégé**

L'invention concerne un chitosane sous forme de poudre micronisée, son utilisation pour lutter contre certaines levures, comme des *Brettanomyces*, et une méthode de traitement d'un liquide alimentaire.

## Description

L'invention concerne un chitosane sous forme de poudre fine et contrôlée, son utilisation pour lutter contre certaines levures, comme des *Brettanomyces,* et une méthode de traitement d'un liquide alimentaire.

### ETAT DE L'ART

Les *Brettanomyces* sont décrits comme un agent de contamination de nombreux produits de fermentation incluant le vin, le cidre, la bière, le kombucha, le kefyr, la téquila, etc.

Dans le milieu viticole, la détection des levures de contamination au vignoble est rendue délicate par le fait qu'elles sont à ce stade minoritaires. Toutefois la fermentation (processus de transformation des sucres en alcool) va entraîner une véritable « sélection » de ces microorganismes. En effet, les *Brettanomyces* sont particulièrement résistantes à l'éthanol et au SO₂ et sont capables de subsister dans le milieu malgré son appauvrissement en sucres. De plus, certaines techniques ou certains protocoles de vinification peuvent favoriser le développement des *Brettanomyces* dans le vin, comme l'élevage sur lies. Pour que les *Brettanomyces* puissent se développer, moins de 500 mg de sucre leur suffisent.

L'impact des levures de contamination *Brettanomyces* a été prouvé par de nombreux auteurs sur des vins dans différents pays, des vins mousseux allemands, des vins jaunes d'Arbois, ou des vins du midi de la France. En 1965, Domercq a rapporté l'isolement des *Brettanomyces* dans des moûts des appellations Saint Emilion et premières côtes de bordeaux et dans des vins rouges ou blancs en cours de conservation des appellations Médoc, Graves et Saint-Emilion. Quant aux cépages, le pinot semble être le plus touché. En Bourgogne, par exemple, pour le millésime 2000, 50% des vins en fermentation issus de ce cépage et 25% après embouteillage avaient été affectés.

En définitive, la maîtrise de cette altération reste difficile, même avec des pratiques oenologiques réfléchies. Les moyens de lutte sont essentiellement préventifs, ils permettent d'agir sur les populations de *Brettanomyces* (SO2, DMDC, flash pasteurisation, filtration). Toutefois ces traitements modifient les qualités organoleptiques des vins et ne préservent pas les vins traités des contaminations ultérieures.

Ce problème n'est pas seulement associé aux vins, mais s'étend aux liquide alimentaire d'origine végétale notamment préparées par fermentation.

Le chitosane est connu comme auxiliaire technologique, mais son utilisation en solution requière des concentrations élevées, généralement de l'ordre de 30 à 100g/hL. D'autre part les propriétés antibactériennes et antifongiques du chitosane ont été largement étudiées et documentées et sont aujourd'hui bien reconnues. Le chitosane est connu pour son effet antimicrobien, dans un temps relativement court, quand il est utilisé en solution avec des acides organiques, en général à pH acide, à des concentrations de l'ordre de 0.5 à 1,5% (soit des concentrations de 0.5g/100ml, 5g/l, 500g/hl à 1000g/hl). Ces concentrations sont élevées car dans la plupart des applications cosmétiques, alimentaires ou médicales on a besoin d'un spectre d'inhibition large (bactéries, levures).

De la même manière, l'activité antifongique du chitosane vis-à-vis de divers champignons excepté ceux contenant du chitosane comme composant important de leur paroi cellulaire est aussi décrite. Le chitosane en solution a été étudié pour lutter contre les souches phytopathogènes *(Fusarium, Rhizopus, Phylium, Candida, Aspergillus).* Le chitosane en solution a également été étudié sur la croissance sur *Saccharomyces cerevisiae* et des bactéries lactiques *(Pediococcus et Lactobacillus*) durant la fermentation. Les auteurs démontrent que le chitosane inhibe de manière plus marqué la croissance de Saccharomyces cerevisiae que des bactéries lactiques *(Pediococcus et Lactobacillus).*

L'action fongicide du chitosane sous la forme de microparticules ayant une taille inférieure à 5µm est décrite par Allan C. and al. (Allan C. and al., (1979) The fungicidal effect of chitosan on fungi of varying cell wall composition Experimental Mycology, 3: 285-287) dans des tests de laboratoire visant à mesurer l'inhibition de la croissance des micro-organismes en présence et en l'absence de chitosane. Les concentrations utilisées sont élevées (0.1 à 6 g/L). Aucune donnée n'est fournie au sujet de l'action contre *Brettanomyces.* De plus les auteurs ont observé que la variabilité de réponse et la sensibilité au chitosane sont fort dépendantes du genre et de l'espèce.

Gomes-Rivas et al. (Gomes-Rivas L. and al., (2004) Selective antimicrobial action of chitosan against spoilage yeasts in mixed culture fermentations, J. Int Microbial Biotechnol 31:16-22) décrivent l'action du chitosane pour lutter spécifiquement contre le genre Brettanomyces/Dekkera. Cependant les auteurs décrivent l'effet du chitosane sous la forme d'une poudre fine de microparticules ayant une taille inférieure à 420µm, DA 9% ; DA= degré d'acétylation) sur la croissance de *Brettanomyces bruxellensis* et sur celle de *Saccharomyces cerevisiae.* Les tests sont réalisés en milieu de culture. L'effet n'est donc pas mesuré sur la matrice vin ce qui rend aléatoire toute conclusion sur l'utilisation d'un tel chitosane comme auxiliaire technologique. Les auteurs indiquent que la croissance de *B. bruxellensis* est inhibée pendant les 80 premières heures de traitement par le chitosane mais que la croissance reprend au-delà de cette période de latence. Ils montrent que le chitosane à 600 g/hl permet de contrôler la croissance de B. *bruxellensis.* Cette concentration reste donc très élevée pour une utilisation industrielle convenable.

La demande de brevet US 2004/0176477 décrit un chitosane micronisé soluble dans l'eau, utilisé comme antimicrobien vis-à-vis de *Malassezia furfur* et *Staphylococcus epidermis.*

La demande de brevet FR 2 599 048 enseigne d'après les exemples que l'on peut utiliser une concentration de chitosane d'environ 80g par hectolitres de liquide alimentaire pour stabiliser ce liquide alimentaire, notamment du point de vue de sa couleur. Cette concentration de chitosane reste élevée.

Le brevet US 6,482,456 décrit une solution aqueuse de chitosane, c'est-à-dire de chitosane soluble dans l'eau.

La demande de brevet FR 1 164 984 enseigne d'utiliser le chitosane en mélange avec la bentonite lorsque la quantité de chitosane doit être diminuée pour la clarification de jus pressés.

Comme déjà mentionnée, sous sa forme cationique, le chitosane est pourvu d'une activité antimicrobienne sur certaines souches. La présence de groupements fonctionnels facilement substituables permet de modifier les propriétés du chitosane et donc son activité. Par exemple, le glutamate ou lactate de chitosane en solution a déjà été utilisé pour limiter une action antimicrobienne ou antifongique.

Cependant le chitosane proposé à ce jour n'est pas utilisé en tant qu'auxiliaire technologique de manière à répondre aux attentes des professionnels de la filière pour la prévention du « risque *Brettanomyces* ».

### BUTS DE L'INVENTION

C'est pour cela que la société Kitozyme SA a mis en place un programme de recherche et développement pour trouver un auxiliaire technologique résolvant les problèmes techniques décrits ci-dessus et ci-dessous.

L'invention a pour but principal de résoudre le nouveau problème technique consistant en la fourniture d'un auxiliaire technologique, pour lutter contre les levures du genre *Brettanomyces.*

L'invention a également pour but de résoudre le nouveau problème technique consistant en la fourniture d'un auxiliaire technologique, pour lutter contre les levures indésirables dans un liquide alimentaire, et en particulier d'un liquide alimentaire préparé par fermentation, et notamment les levures du genre *Brettanomyces.*

L'invention a aussi pour but de fournir un auxiliaire technologique utilisable fiable du point de vue de la sécurité alimentaire et ne dégradant pas la qualité des liquides alimentaires traités.

L'invention a pour but de résoudre ces problèmes techniques à l'échelle industrielle, notamment pour l'industrie des liquides alimentaires, éventuellement alcoolisés et/ou fermentés.

### DESCRIPTION DE L'INVENTION

Ainsi, la présente invention concerne un chitosane sous forme de poudre dont la granulométrie (diamètre des particules) est comprise entre 0.1 et 200 µm.

Avantageusement, le chitosane comprend un granulométrie comprise entre 5 et 100 microns, et de préférence entre 5 et 60 microns, et encore de préférence comprise entre 5 et 50 microns.

Selon une variante, le chitosane comprend une granulométrie inférieure à 70 microns, et de préférence inférieure à 50 microns.

De manière surprenante, une poudre ayant une granulométrie inférieure à 100 microns permet d'obtenir un effet très important et très rapide sur la population des levures indésirables. Cet effet est particulièrement marqué lorsque la granulométrie est inférieure à 50 microns.

La granulométrie est celle obtenue par diffractométrie au laser.

Le chitosane de l'invention a de préférence une granulométrie présentant un D(0.5) (diamètre médian de la distribution totale avec 50% du volume des particules ont un diamètre inférieur à cette valeur et 50% ont un diamètre supérieur) comprise entre 5 et entre 30µm, de préférence entre 10 et 30µm, et encore de préférence entre 10 et 25µm. D(0.5) représente donc le milieu de la distribution compte tenu des aires sous la courbe de répartition de la granulométrie.

Selon une variante, le chitosane peut être utilisé après tamisage.

De préférence, le chitosane comprend un degré d'acétylation (DA) compris entre 0 et 30 mol%. Le degré d'acétylation est le rapport du nombre d'unités N-acétyl-glucosamine sur le nombre de monomères totaux. Le degré d'acétylation du chitosane est déterminé par titration potentiométrique qui consiste à déterminer le degré d'acétylation du chitosane par titration des groupes amines. Elle est basée sur les travaux de Rinaudo et al.,(1999). Brièvement, le chitosane est mis en solution dans un excès d'acide chlorhydrique dilué. Les groupements amines (sur les unités de glucosamine non acétylées (G)) sont chargées positivement (HCl en excès)). La solution est ensuite titrée avec une solution de NaOH diluée, à l'aide d'un titrateur automatique (KEM, automatic potentiometric titrator, AT-500N) et le pH est mesuré. Dans la première partie de la réaction, on détermine la quantité d'HCl en excès. Ensuite, on détermine la quantité de groupements amines chargés :
La courbe de titrage montrent deux points d'inflexion. La différence entre les deux volumes de NaOH permet de connaître la quantité d'amines libres.

Le chitosane est référencé sous le N°CAS 9012-76-4 Le chitosane de l'invention est un polysaccharide préparé à partir d'une origine fongique. Il est extrait et purifié à partir de sources fongiques alimentaires ou biotechnologique sûres et abondantes tels que *Agaricus bisporus* ou *Aspergillus niger.* Le chitosane est obtenu par hydrolyse d'un extrait riche en chitine. La chitine est un polysaccharide composé de plusieurs unités N-acétyl-Dglucosamine reliées entre elles par une liaison de type ß (1,4). Le chitosane est constitué des unités sucres glucosamine (unités désacétylées) et d'unités N-acétyl-D-glucosamine (unités acétylées) reliées entre elles par des liaisons de type ß (1,4) et constitue un polymère du type Poly(N-acetyl-D-glucosamine)-poly(D-glucose). Dans l'invention, le pourcentage de glucans (unités D-glucoses) est de préférence inférieur à 15% (masse/masse) du polymère.

Le chitosane de l'invention est utile comme auxiliaire technologique. Un auxiliaire technologique s'entend d'une substance ou matière à l'exclusion de tout appareil ou instrument, qui n'est pas consommée comme ingrédient alimentaire en soi, qui est intentionnellement utilisée dans la transformation des matières premières, des denrées alimentaires ou de leurs ingrédients, pour répondre à un certain objectif technologique pendant le traitement ou la transformation et pouvant avoir pour résultat la présence non intentionnelle mais inévitable de résidus ou de denrées dans le produit fini (définition du CODEX Alimentarius).

Le chitosane de l'invention est avantageusement d'origine fongique, et de préférence issus du mycelium d'un champignon du type *Ascomycète,* et en particulier de *Aspergillus niger,* et/ou d'un champignon *Basidiomycète,* et en particulier *Lentinula edodes* (shiitake) et/ou *Agaricus bisporus.* De préférence le champignon est *Aspergillus niger.* Le chitosane peut être d'origine OGM, mais de préférence est d'origine non-OGM. Tout type de chitosane peut être utilisé. Une méthode de préparation du chitosane est celle décrite dans les brevets WO03068824 (EP1483299 ; US 7,556,946).

De nombreuses études démontrent que l'activité antimicrobienne du chitosane varie en fonction du degré acétylation (DA) et de la masse moléculaire (Masse Moléculaire : MW) du chitosane. L'invention courve ainsi les différents domaines de DA et MW.

Les deux paramètres (DA et MW) affectent l'activité antimicrobienne du chitosane indépendamment, toutefois il semblerait que l'influence du MW soit plus importante sur l'activité antimicrobienne que l'influence du DA (Sekiguchi, S., and al. (1994) Molecular weight dependency of antimicrobial activity by chitosan oligomers, in: Nishinari, K. & Doi, E. (Eds), "Food Hydrocolloids: Structures, Properties and Functions", Plenum Press, New York).

Pour citer des exemples récents, des études effectuées sur les bactéries dont notamment par Omura, Y., and al. (Omura, Y., and al. (2003) Antimicrobial activities of chitosan with different degrees of acetylation and molecular weights Biocontrol. Sci., 8(1) : 25-30); Tsai, G. J.; Zhang, S. L., Shieh, P. L. (Tsai, G. J. et al. (2004) Antimicrobial Activity of a Low-Molecular-Weight Chitosan Obtained from Cellulase Digestion of Chitosan J. Food Prot., 67(2) : 396-398); Zivanovic S., et al. (Zivanovic S. et al., (2004) Molecular Weight of Chitosan Influences Antimicrobial Activity in Oil-in-Water Emulsions J. Food Prot., 67(5): 952-959.

Pour les champignons, Eaton, P. And al. (Eaton, P. And al. , (2008) Atomic force microscopy study of the antibacterial effects of chitosans on Escherichia coli and Staphylococcus aureus Ultramicroscopy, 108 (10): 1128-1134.) ont trouvé que plus la masse moléculaire (MW) du chitosane est faible meilleur est l'effet inhibiteur du chitosane sur la croissance et la multiplication des microorganismes.

De la même façon, de nombreuses études ont démontré une relation inverse entre le DA du chitosane et son activité antimicrobienne (Hongpattarakere, T., and al. (2008) Effect of deacetylation conditions on antimicrobial activity of chitosans prepared from carapace of black tiger shrimp (Penaeus monodon) Songklanakarin J. Sci. Technol.. 30(1): p.1-9; Tsai, G. J., and al. (2002) Antimicrobial activity of shrimp chitin and chitosan from different treatments Fisheries Sci., 68 (1): 170-177).

L'invention concerne également une composition comprenant un chitosane tel que défini précédemment.

En particulier l'invention concerne une suspension de chitosane de l'invention. Plus particulièrement cette suspension est adaptée pour un utilisation en tant qu'auxiliaire technologique, par exemple du point de vue de sa composition qualitative, de sa pureté, et/ou de la source du chitosane.

Le chitosane est incorporé sous forme de poudre dans le liquide alimentaire à traiter. Il est notamment utilisé en vue d'une stabilisation microbiologique par élimination des *Brettanomyces.*

Le chitosane de l'invention est donc insoluble dans l'eau. On entend par « insoluble dans l'eau » un chitosane dont au moins 90 %, et de préférence 95 % (masse/masse), n'est pas soluble dans l'eau distillée. Dans les exemples, le chitosane étant considéré totalement insoluble on peut assimiler le pourcentage de matières solubles dans l'eau à des impuretés.

Le chitosane de l'invention, insoluble, possède une partie cristalline, généralement supérieure à 2% (masse/masse) après hydratation.

Avant son utilisation en tant qu'auxiliaire technologique, le chitosane est suspendu et mélangé dans un liquide (comme par exemple l'eau) compatible avec le liquide alimentaire à traiter, c'est-à-dire qu'il n'est pas préjudiciable à l'utilisation finale du liquide alimentaire. Le rapport massique chitosane/liquide (par exemple : chitosane/eau) est de préférence compris entre 1/1 et 1/10, et de préférence de 1/5 (masse/masse ; m/m). Le chitosane ne se dissout pas dans l'eau, il faut donc bien brasser au préalable le mélange. De préférence le mélange est brassé juste avant son ajout au liquide à traiter.

Le chitosane ou suspension de chitosane de l'invention est ajoutée au liquide alimentaire à traiter. Le chitosane doit être également homogène sur l'ensemble du volume de liquide à traiter et par exemple au cours d'un remontage en versant le produit par petits volumes, pour assurer une bonne répartition dans la masse du vin. Le chitosane doit donc être additionné progressivement. Il est préférable d'homogénéiser le liquide à traiter en même temps que l'ajout du chitosane. Pour le traitement d'un vin, le remontage complet de la cuve est généralement nécessaire. A l'issue du traitement, le chitosane (auxiliaire technologique) est éliminé par soutirage pour éliminer le chitosane et les microorganismes absorbés. On réalise cette étape d'élimination par exemple après un temps d'action de 7 jours à 10 jours et après décantation naturelle du liquide à traiter, et typiquement dans le traitement d'un liquide alimentaire fermenté comme un vin.

L'invention concerne également l'utilisation de chitosane ou d'une composition tel que défini précédemment, en tant qu'antifongique, et notamment pour lutter contre les levures, en particulier des levures Brettanomyces et/ou Dekkera, en encore plus particulièrement des levures *B. anomalus, B. bruxellensis, B. claussenii, B. custersianus, B. lambicus, B. naardenensis, et*/*ou B. nanus.* L'action du chitosane de l'invention n'est pas limitée à une souche particulière car plusieurs souches du même type ont été testées. Comme indiqué précédemment, les concentrations de chitosane de l'art antérieur connus sont élevées car dans la plupart des applications cosmétiques, alimentaires ou médicales on a besoin d'un spectre d'inhibition large (bactéries, levures). Or cet effet n'est pas recherché dans la présente invention, au contraire il faut éviter l'inhibition des bactéries et des levures utiles par exemple lors d'un fermentation comme dans l'élevage des vins par exemples. L'invention est donc surprenante car à dose très faible (<10g/hl), le chitosane de l'invention agit de manière sélective sur les levures indésirables.

L'invention concerne d'autre part l'utilisation de chitosane ou d'une composition tel que défini précédemment, en tant qu'antifongique, et notamment pour lutter contre les levures indésirables dans un liquide alimentaire, et notamment d'origine végétale, c'est-à-dire préparé à partir de plantes (raisin, pommes, pomme de terre, betteraves, etc), et en particulier d'un liquide alimentaire préparée par fermentation, par exemple un vin, une bière, un cidre, un vin effervescent, ou un jus de fruit. L'invention concerne le traitement des liquides alimentaires tels que l'eau, les boissons fermentées (boissons obtenues par transformation des sucres en solution en alcool, par fermentation du produit de base), les vins de liqueur (moût dans lequel on ajoute un alcool) les boissons spiritueuses (obtenues par la distillation de boissons fermentées). Les Vins De liqueur le plus courants sont: le Pineau des Charentes (à base de Cognac), le Floc de Gascogne (à base d'Armagnac), le Ratafia de Champagne et de Bourgogne, le Riquiqui, le Macvin du Jura, la Cataroise de Béziers, le Pommeau de Normandie (à base de Calvados). Les liquides alimentaires peuvent être obtenus par différentes parties de plantes : feuilles, racines, céréales, fruit, etc. L'invention concerne donc tous liquides alimentaires, y compris tous liquides susceptibles d'être contaminés par des levures indésirables de type Brettanomyces ; les liquides issus de fermentation (alimentaires ou non), comme le moût avant fermentation ou en cours de fermentation, ou du liquide fermenté (par exemple moût/biomasse de végétaux - pomme de terre, betteraves, ... - ou de raisins) ; les liquides alimentaires d'origine végétale non fermentés (jus de fruits), et les liquides alimentaires issus de la fermentation des moûts de raisin.

Le traitement de l'invention est donc notamment applicable au pisco, Pineau des Charentes, eaux de vies, cidre, bière, alcool à destination industriel et vin.

Il a été découvert de manière surprenante que le chitosane de l'invention peut être utilisé en tant qu'antifongique contre les levures indésirables dans un liquide alimentaire, et notamment d'origine végétale, éventuellement fermentée qui est généralement complexe du point de vue de sa composition. La capacité du chitosane à éliminer des levures, notamment de celles des genres ou espèces citées ci-dessus au sein d'un liquide alimentaire végétale, de surcroit obtenu ou encore en fermentation n'était pas prédictible.

Selon un autre aspect, l'invention concerne une méthode de traitement curatif et/ou préventif d'un liquide alimentaire, et notamment d'origine végétale, et en particulier d'un liquide alimentaire d'origine végétale préparé par fermentation, dans lequel des levures indésirables sont à éliminer, dont la présence est à prévenir, et/ou dont la population est à limiter, et en particulier de levures du genre *Brettanomyces* et/ou *Dekkera,* en encore plus particulièrement des levures *B. anomalus, B. bruxellensis, B. claussenii, B. custersianus, B. lambicus, B. naardenensis, et*/*ou B. nanus,* ladite méthode comprenant la mise en présence du liquide alimentaire avec un chitosane ou d'une composition tel que défini précédemment.

L'invention concerne aussi une méthode d'hygiène et de prévention dans un local de production (usine de production, chais, etc.), qui permet notamment d'éviter un arrêt de la production pour nettoyage. L'usage préventif est important d'une manière générale (hygiène préventive du chais ou de l'usine), autant que le traitement curatif.

Le liquide alimentaire à traiter selon l'invention est en particulier un vin, une bière, ou un cidre.

Les levures indésirables sont notamment *B. anomalus et*/*ou B. bruxellensis.* L'invention concerne en particulier une méthode de traitement d'un liquide alimentaire, éventuellement fermentée, pour lutter contre la présence de levures du genre *Brettanomyces*, et en particulier de l'espèce *Brettanomyces bruxellensis.*

Selon une variante préférée le chitosane est présent à une concentration de 1 à 10 g/hL de liquide alimentaire à traiter, et de préférence compris entre 2 et 5g/hL de liquide alimentaire à traiter. La concentration utilisée est donc particulièrement faible en comparaison aux autres produits de l'art antérieur, qui nécessitent souvent plus de 20g/hL comme pour un traitement avec le dicarbonate de diméthyle (DMDC).

Le liquide alimentaire est mise en présence de chitosane pendant une période de temps suffisante pour éliminer, limiter, ou prévenir la présence de levures, notamment des levures décrites ci-dessus. Cette période de temps est généralement d'au moins 3 jours, et de préférence d'au moins 5, et encore de préférence d'au moins 7 jours.

On entend par « éliminer » la présence des levures, le fait de réduire la population de levures indésirables en dessous du seuil de détection (10UFC/mL). Par « limiter » on entend limiter la population d'au moins un facteur 10 voire 100 la population de levure indésirables. Par « prévenir » on entend empêcher le développement, la prolifération et/ou limiter la population de levure indésirables.

Le traitement peut être effectué sur un liquide alimentaire en cours de fermentation. En effet, il a été découvert de manière surprenante que le chitosane de l'invention permet de continuer une fermentation, et notamment une fermentation alcoolique et/ou malolactique. La fermentation n'est pas perturbée par la présence du chitosane de l'invention. Le chitosane de l'invention n'a notamment pas d'effet néfaste sur une population de *Saccharomyces cerevisiae.*

Le chitosane et/ou la composition comprenant le chitosane, est de préférence séparée, par exemple par sous-tirage, collage, filtration et/ou centrifugation du liquide alimentaire pour conserver le liquide alimentaire. Le liquide alimentaire peut être soutiré après un temps de contact suffisant avec le chitosane de l'invention.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en poids, sauf indication contraire, et la température est exprimée en degré Celsius sauf indication contraire, et la pression est la pression atmosphérique, sauf indication contraire.

### EXEMPLES

### A- Les souches de levure

Les souches de *Brettanomyces bruxellensis* utilisées pour l'inoculation artificielle du vin sont des souches identifiées comme majoritaires sur le vignoble méditerranéen et ont des génotypes 100% A et 100% B. Elles proviennent de la collection des souches ICV-ADN ^{id.} Elles sont conservées à 4°C sur milieu gélosé incliné (milieu YAC) après 7 jours d'incubation à 28°C.

### B- Le milieu et les conditions de culture du levain

Le levain est un milieu d'acclimatation dans lequel les levures peuvent croître et atteindre une population de l'ordre de 10⁷UFC/ml, tout en s'adaptant à un environnement peu favorable (pH acide, taux en éthanol élevé)

La composition du levain utilisé pour les expérimentations est décrite dans le Tab. 1 suivant :

**Tableau 1- Composition du milieu levain pour Brettanomyces bruxellensis**

| Composés | Concentrations/conditions |
|---|---|
| Yeast Nitrogen Base | 6,7g/l |
| Glucose | 20g/l |
| Ethanol | 10% (v/v) |
| Eau déminéralisée | Compléter au volume souhaité |
| pH | Ajuster à 3,5 avec des cristaux d'acide tartrique |
| Stérilisation | 20 min à 121 °C |

### C- Techniques analytiques de dénombrement

Le dénombrement des *Brettanomyces* viables s'est fait par mise en culture sur milieu gélosé spécifique YAC (YEPD + Actidione + Chloramphénicol) selon le protocole suivant : les échantillons sont prélevés et dilués en cascade sous environnement stérile, dans des tubes contenant 9ml ou 9,9ml d'eau physiologique stérile. 100µl sont mis en culture par étalement sur milieu gélosé YAC, à partir de la dilution appropriée pour avoir entre 30 et 300 colonies sur la gélose. Les boîtes sont incubées à 28°C pendant 10 jours. Les mises en culture sont doublées.

### D- Micronisation du chitosane

Le chitosane est obtenu par extraction et désacétylation (hydrolyse des groupements N-acétyl-glucosamine) au départ du mycélium d*'Aspergillus niger* par action de la soude. Après plusieurs étapes de lavage et purification, le chitosane est ensuite séché et broyé pour atteindre la granulométrie désirée. On peut également utiliser un chitosane insoluble dans l'eau commercialement disponible pour ensuite le microniser. Le chitosane micronisé résultant doit être insoluble dans l'eau conformément à l'invention.

La micronisation du chitosane issu de source fongique *(Aspergillus niger*) est réalisée à l'aide d'un broyeur à jets d'air opposés. Différentes marques et modèles d'équipements peuvent être utilisés selon les quantités de poudre à traiter : par exemple équipement de la marque Hozokawa - Alpine (modèle 200 AFG) pour les quantités >200kg, et par exemple équipement de la marque Netzsch - modèle CGS 10 pour les plus petites quantités.

La poudre est broyée en continu jusqu'au passage de la poudre micronisée à travers une turbine de sélection pour l'obtention d'une poudre dont les particules ont un diamètre inférieure à 100 µm; voir à 50µm. Une analyse granulométrique de la poudre est ensuite réalisée à l'aide d'un diffractomètre Laser Mastersizer 2000 de chez Malvern Instruments Ltd.

Le D(0.50) est compris entre 10 et 30µm et de préférence entre 10 et 25µm, voire entre 10 et 20µm.

### E- Caractéristiques des lots de chitosane utilisés dans les exemples 1-26

**Tableau 1**

| Exemple | DA (% mol) | Granulométrie (par diffractomètre Laser) | Granulométrie (par tamisage) | Contenu en chitosane (%)* | Glucans résiduels (%) |
|---|---|---|---|---|---|
| 2, 3 | 22 | ND | <50µm | 89.6 | 6.7 |
| 1.1 | ND | ND | ND | ND | ND |
| 1.2, 10-15 | 4.2 | <50µm ; D(0.5) : 13.6µm | ND | 87 | 5.2 |
| 4 | 22 | ND | <25µm, <50µm, <90µm | 89.6 | 6.7 |
| 5 | 22 8.5 | ND | ND | 89.6 90.8 | 6.7 7.6 |
| 6.1 | 8.5 | ND | <25µm, <90µm | 90.8 | 7.6 |
| 6.2 | 22 | ND | <25µm, <90µm | 89.6 | 6.7 |
| 7-9 ; 16 | 22 | ND | <50µm | 89.6 | 6.7 |
| 17 | 4.2 | D(0.5) : 13.6 | <50µm | 87 | 5.2 |
| 18 | 4.2 | D(0.5) : 13.6 | <50µm | 87 | 5.2 |
| 19 | 14.30 | D(0.5) :14.1 | <50µm | 87 | 10.86 |
| 20 | 11.80 | D(0.5) :14.0 | <50µm | 90 | 8.3 |
| 21 | 11.80 | D(0.5) :14.0 | <50µm | 90 | 8.3 |
| 22 | 12.47 | D(0.5) :13.6 | <50µm | 88 | 10.21 |
| 23 | 14.49 | D(0.5) :11.2 | <50µm | 87 | 7.77 |
| 26 | 11.80 | D(0.5) :14.0 | <50µm | 90 | 8.3 |

| | | | | | |
|---|---|---|---|---|---|
| *calculé en soustrayant les cendres, protéines et glucans au poids de l'échantillon sec. ND : Non déterminé. Un certain pourcentage de glucans résiduels subsistent dans le chitosane. | | | | | |

### EXEMPLE 1.1- Traitement d'un vin rouge contaminé en Brettanomyces bruxellensis à l'aide chitosane à différentes doses (essai laboratoire)

Un vin fini du Médoc (Merlot) millésime 2006 présentant un niveau de population initiale en *Brettanomyces bruxellensis* de 2,8 10⁵ cellules/ml a été traité par ajout de chitosane à différentes doses.

**Tableau 2- Dénombrement par PCR quantitative des Brettanomyces bruxellensis 7 jours après traitement**

| Dose de traitement | Témoin | 0,2g/hl | 0,5g/hl | 0,7g/hl | 1g/hl | 2g/hl | 5g/hl |
|---|---|---|---|---|---|---|---|
| Dénombrement des *Brettanomyces* | 2,8 10⁵ | 3563 | 1527 | 509 | 254 | 0 | 0 |

Après 7 jours de traitement, le chitosane à la dose de 2g/hl permet une élimination totale des *Brettanomyces bruxellensis* présentes dans le vin.

### EXEMPLE 1.2- Traitement d'un vin rouge contaminé en Brettanomyces intermedius à l'aide chitosane à différentes doses (essai laboratoire)

Un vin fini du millésime 2009 présentant un niveau de population initiale en *Brettanomyces intermedius* de 3,7 10⁶ cellules/ml a été traité par ajout de chitosane (DA≈4,2mol%) à différentes doses. Le vin témoin ne subit aucun traitement.

**Tableau 3- Dénombrement par PCR quantitative et sur milieu gélosé spécifique des Brettanomyces intermedius 10 jours après traitement**

| Dose de traitement | Témoin | 0 g/hl | 2 g/hl | 3 g/hl | 4 g/hl |
|---|---|---|---|---|---|
| Dénombrement des *Brettanomyces* (q-PCR) | 3,7 10⁶ | 8,8 10⁶ | 2,3 10² | 1,0 10³ | 30 |
| Dénombrement des *Brettanomyces* (milieu gélosé) | 1,1 10⁶ | 1,8 10⁶ | 30 | <1 | 1 |

Après 10 jours de traitement, le chitosane permet une diminution importante des *Brettanomyces intermedius* présentes dans le vin à la dose de 4g/hl.

### EXEMPLE 2- Traitement d'un vin rouge contaminé en Brettanomyces bruxellensis de souche A à l'aide de chitosane à la dose de 4 g/hl (essai laboratoire)

Un vin fini du Languedoc Roussillon (Merlot) millésime 2008 présentant un niveau de population initiale en *Brettanomyces bruxellensis* de souche A de 10⁶ cellules/ml a été traité par ajout de chitosane à la dose de 4 g/hl ou par ajout de DMDC à la dose de 20g/hl. Le vin témoin ne subit aucun traitement.

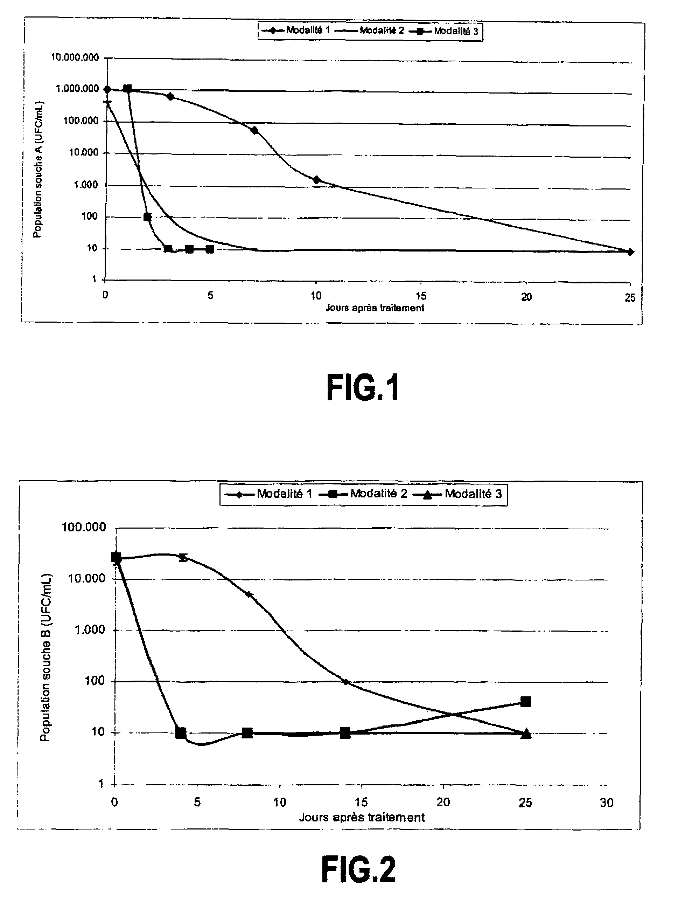

**Figure 1****-** Suivi de la population en *Brettanomyces bruxellensis* souche A dans le vin après le traitement
**Légende -**
**Modalité 1** : Témoin ;
**Modalité 2** : Traitement à l'aide de DMDC 20g/hl ;
**Modalité 3** : Traitement à l'aide de chitosane 4g/hl

On constate que le traitement à l'aide de chitosane (modalité 3) est aussi efficace que le traitement à l'aide de DMDC (modalité 2) vis-à-vis des *Brettanomyces bruxellensis* souche A. En effet, on constate une réduction significative de la population en *Brettanomyces bruxellensis* souche A (≤ 10UFC/ml) en 5 à 10 jours avec les deux types de traitement.

### EXEMPLE 3- Traitement d'un vin rouge contaminé en Brettanomyces bruxellensis de souche B à l'aide de chitosane à la dose de 4 g/hl (essai laboratoire)

Un vin fini du Languedoc Roussillon (Merlot) millésime 2008 présentant un niveau de population initiale en *Brettanomyces bruxellensis* de souche B de 10⁵ cellules/ml a été traité par ajout de chitosane à la dose de 4 g/hl ou par ajout de DMDC à la dose de 20g/hl. Le vin témoin ne subit aucun traitement.

Figure 2- Suivi de la population en *Brettanomyces bruxellensis* souche B dans le vin après le traitement
**Légende -**
**Modalité 1** : Témoin ;
**Modalité 2** : Traitement à l'aide de DMDC 20g/hl ;
**Modalité 3 :** Traitement à l'aide de chitosane 4g/hl

On constate que le traitement à l'aide de chitosane (modalité 3) est aussi efficace que le traitement à l'aide de DMDC (modalité 2) vis-à-vis des *Brettanomyces bruxellensis* souche B. En effet, on constate une réduction significative de la population en *Brettanomyces bruxellensis* souche B (≤ 10UFC/ml) en 5 à 10 jours avec les deux types de traitement.

### EXEMPLE 4- Traitement d'un vin rouge contaminé en Brettanomyces bruxellensis à l'aide de chitosane de différentes granulométries à la dose de 4 g/hl (essai laboratoire)

Un vin fini du Languedoc Roussillon (Merlot) millésime 2008 présentant un niveau de population initiale en *Brettanomyces bruxellensis* de 10⁵ cellules/ml a été traité par ajout de chitosane de différentes granulométries : < 25µm, < 50µm, < 90µm à la dose de 4g/hl. Le vin témoin ne subit aucun traitement.

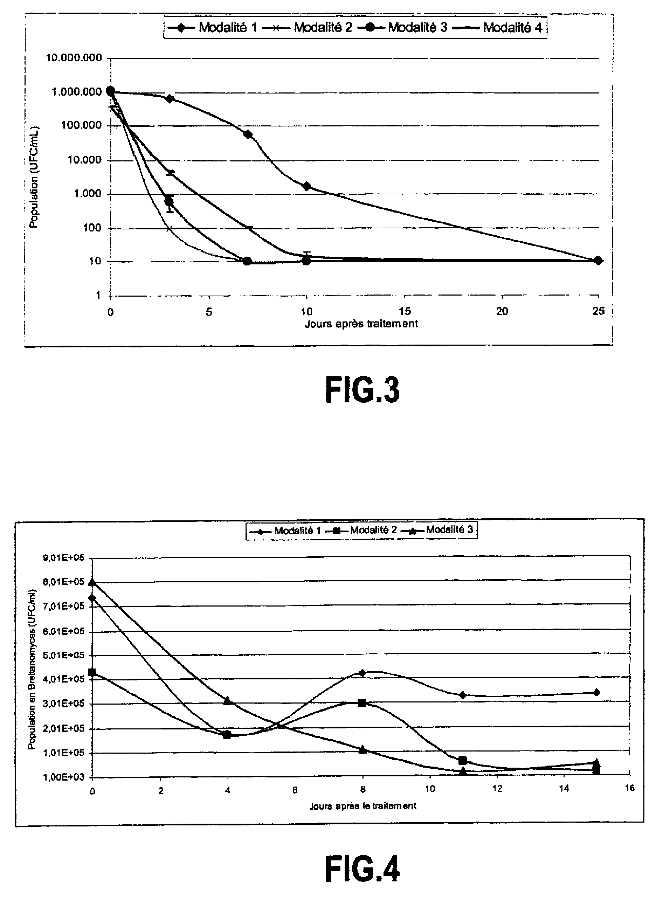

**Figure 3****-** Suivi de la population en *Brettanomyces bruxellensis* dans le vin après le traitement
**Légende** -
**Modalité 1** : Témoin ;
**Modalité 2** : Traitement au chitosane 4g/hl - granulométrie < 25µm ;
**Modalité 3** : Traitement au chitosane 4g/hl - granulométrie <50µm;
**Modalité 4** : Traitement au chitosane 4g/hl - granulométrie <90µm

Après 7 jours de traitement, l'utilisation du chitosane de granulométrie <25µm (modalité 2) et de granulométrie < 50µm (modalité 3) engendre une diminution de la population en *Brettanomyces bruxellensis* d'un facteur 5000 à 10000 pour atteindre le seuil de détection (≤10 UFC/ ml).

Le chitosane présentant une granulométrie < 90µm (modalité 4) a permis de réduire la population en *Brettanomyces bruxellensis* au seuil de détection (≤10 UFC/ ml) en 10 jours, soit 3 jours de plus que les autres modalités.

### EXEMPLE 5- Traitement d'un vin rouge contaminé en Brettanomyces bruxellensisà l'aide chitosane de différents degrés d'acétylation à la dose de 2 g/hl (essai laboratoire)

Un vin fini du Languedoc Roussillon (Cabernet Sauvignon) millésime 2008 présentant un niveau de population initiale en *Brettanomyces bruxellensis* de 5.10⁵ cellules/ml a été traité par ajout de chitosane de différents degrés d'acétylations : 8,5mol%, 22,0mol% à la dose de 2g/hl. Le vin témoin ne subit aucun traitement.

**Figure 4****-** Suivi de la population en *Brettanomyces bruxellensis* dans le vin après le traitement
**Légende** -
**Modalité 1** : Témoin ;
**Modalité 2** : Traitement au chitosane 2g/hl - DA 22mol% ;
**Modalité 3** : Traitement au chitosane 2g/hl - DA 8,5mol% ;

Après 11 jours de traitement, l'utilisation du chitosane de DA 22mol% (modalité 2) et de DA 8,5mol% (modalité 3) engendre une diminution de la population en *Brettanomyces bruxellensis* d'un facteur 100.

### EXEMPLE 6.1- Traitement d'un vin rouge contaminé en Brettanomyces bruxellensis à l'aide de chitosane de différentes granulométries à la dose de 4 g/hl (essai laboratoire)

Un vin fini du Languedoc Roussillon (Merlot) millésime 2008 présentant un niveau de population initiale en *Brettanomyces bruxellensis* de 10⁵ cellules/ml a été traité par ajout de chitosane (DA≈8,5mol%) de différentes granulométries : <25µm, <90µm à la dose de 4g/hl. Le vin témoin ne subit aucun traitement.

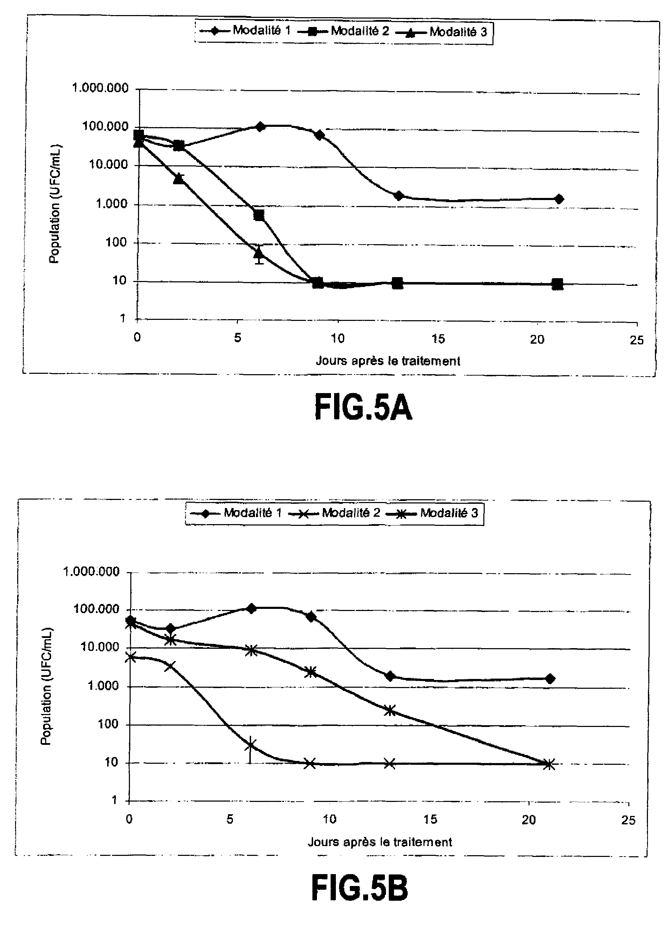

Figure 5A Suivi de la population en *Brettanomyces bruxellensis* dans le vin après le traitement
**Légende -**
**Modalité 1** : Témoin ;
**Modalité 2** : Traitement au chitosane 4g/hl - granulométrie < 25µm ;
**Modalité 3** : Traitement au chitosane 4g/hl - granulométrie <90µm;

Après 9 jours de traitement, l'utilisation des lots de chitosane présentant une granulométrie< 25µm (modalité 2) ; < 90µm (modalité 3) ont permis de faire chuter la population en *Brettanomyces bruxellensis* au seuil de détection (≤10 UFC/ ml) alors que la population du témoin non traité (modalité 1) se maintient dans le même laps de temps à son niveau initial (10⁵UFC/ml).

### EXEMPLE 6.2- Traitement d'un vin rouge contaminé en Brettanomyces bruxellensis à l'aide de chitosane de différentes granulométries à la dose de 4 g/hl (essai laboratoire)

Un vin fini du Languedoc Roussillon (Merlot) millésime 2008 présentant un niveau de population initiale en *Brettanomyces bruxellensis* de 10⁵ cellules/ml a été traité par ajout de chitosane (DA≈22mol%) de différentes granulométries : < 25µm, < 90µm à la dose de 4g/hl. Le vin témoin ne subit aucun traitement.

Figure 5B- Suivi de la population en *Brettanomyces bruxellensis* dans le vin après le traitement
**Légende -**
**Modalité 1** : Témoin ;
**Modalité 2** : Traitement au chitosane 4g/hl - granulométrie < 25µm ;
**Modalité 3** : Traitement au chitosane 4g/hl - granulométrie <90µm;

Après 9 jours de traitement, l'utilisation du lot de chitosane présentant une granulométrie< 25µm (modalité 2) a permis de faire chuter la population en *Brettanomyces bruxellensis* au seuil de détection (≤10 UFC/ ml) alors que la population du témoin non traité (modalité 1) se maintient dans le même laps de temps à son niveau initial (10⁵UFC/ml).

Le chitosane présentant une granulométrie< 90µm (modalité 3) a permis de réduire la population en *Brettanomyces bruxellensis* au seuil de détection (≤10 UFC/ ml) en 21 jours, soit 12 jours de plus que la modalité 2.

### EXEMPLE 7- Efficacité du traitement à l'aide de chitosane à la dose de 4g/hl d'un vin rouge dans lequel la durée d'implantation des Brettanomyces bruxellensisest variable (essai laboratoire)

Un vin fini du Languedoc Roussillon (Merlot) millésime 2008 dont les *Brettanomyces bruxellensis* sont implantées dans le vin depuis 3 jours ou 24 jours ont été traités par ajout de chitosane à la dose de 4g/hl.

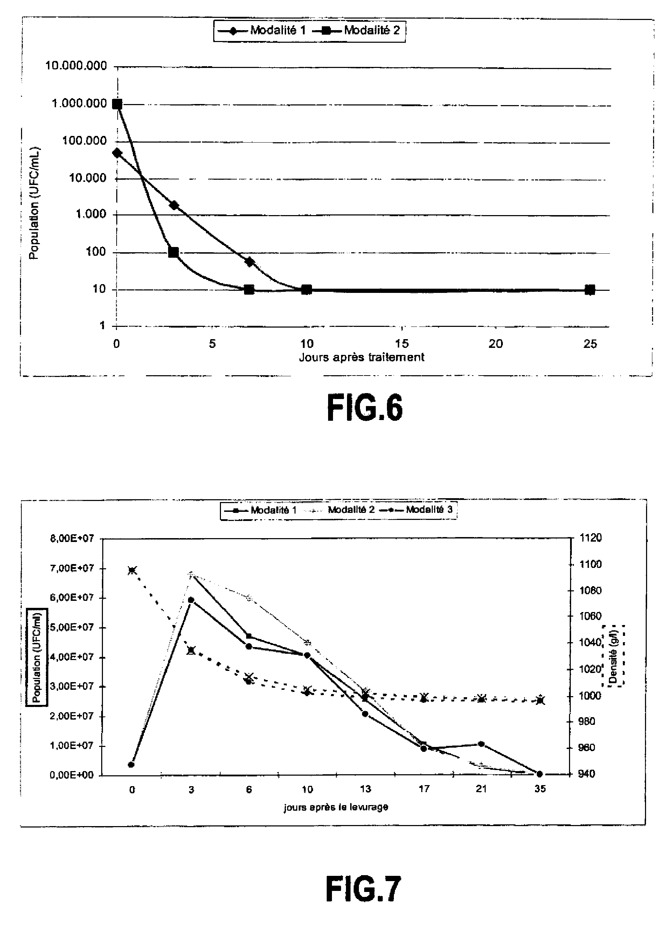

**Figure 6****-** Suivi de la population en *Brettanomyces bruxellensis* dans le vin après le traitement
**Légende -**
**Modalité 1 :** Traitement au chitosane 4g/hl sur une population en *Brettanomyces bruxellensis* implantée depuis 3 jours;
**Modalité 2 :** Traitement au chitosane 4g/hl sur une population en *Brettanomyces bruxellensis* implantée depuis 24 jours;

Cet exemple permet de démontrer que l'impact du chitosane vis-à-vis des *Brettanomyces bruxellensis* est dépendant de leur durée d'implantation dans le vin. Ainsi les populations en *Brettanomyces bruxellensis* implantées depuis 24 jours dans le vin (modalité 2) sont plus sensibles au chitosane. Au bout de 7 jours, la population en *Brettanomyces bruxellensis* a diminué pour atteindre le seuil de détection (≤10 UFC/ ml) alors qu'il faut 10 jours pour atteindre ce même résultat sur une population en *Brettanomyces bruxellensis* fraîchement implantée (modalité 1).

### EXEMPLE 8- Impact de l'utilisation du chitosane à la dose de 4g/hl sur les Saccharomyces cerevisiae (levures importantes dans le processus de fermentation alcoolique des vins) (essai laboratoire)

Un jus de raisin blanc de la marque Casino a été supplémenté en sucre pour atteindre la teneur de 200g/l et en azote pour atteindre une teneur supérieure 200mg/l puis inoculé en *Saccharomyces cerevisiae* ICV D47^{®} à la dose de 30g/hl.

Le chitosane a été ajouté en cours de fermentation alcoolique à deux moments différents :
- densité de 1035 : 3 jours après le levurage
- densité de 1015 : 6 jours après le levurage

Le moût témoin ne subit aucun traitement.

**Figure 7****-** Evolution de la population en *Saccharomyces cerevisiae* et suivi de la densité dans le moût avant et après le traitement au chitosane
**Légende** -
**Modalité 1** : Moût témoin
**Modalité 2** : Traitement au chitosane 4g/hl - 3 jours après le levurage (densité 1035);
**Modalité 3** : Traitement au chitosane 4g/hl - 6 jours après le levurage (densité 1031)

La cinétique de croissance de la population de *Saccharomyces cerevisiae* atteint sa valeur maximale de l'ordre de 6.10⁷UFC/ml après 3 jours de fermentation, puis le nombre de levures se maintient à 5.10⁷UFC/ml pendant 10 jours pour finalement amorcer sa phase de décroissance après 13 jours de fermentation. Cette cinétique est similaire pour toutes les modalités. Par conséquent nous pouvons conclure que le chitosane n'a pas d'impact négatif sur les levures *Saccharomyces cerevisiae.*

### EXEMPLE 9- Impact de l'utilisation du chitosane à la dose de 4g/hl sur les Oenococcus oeni (bactéries importantes dans le processus de fermentation malolactique des vins) (essai laboratoire)

Un vin rouge du Languedoc Roussillon (Merlot) millésime 2008 en fin de fermentation alcoolique a été inoculé ou non en *Oenococcus oeni* Elios 1 ^{®}

Le chitosane a été ajouté soit dans le cadre d'une fermentation malolactique en mode spontanée, soit dans le cadre d'une fermentation malolactique par inoculation.

Le vin témoin ne subit ni inoculation en *Oenococcus oeni* ni traitement au chitosane.

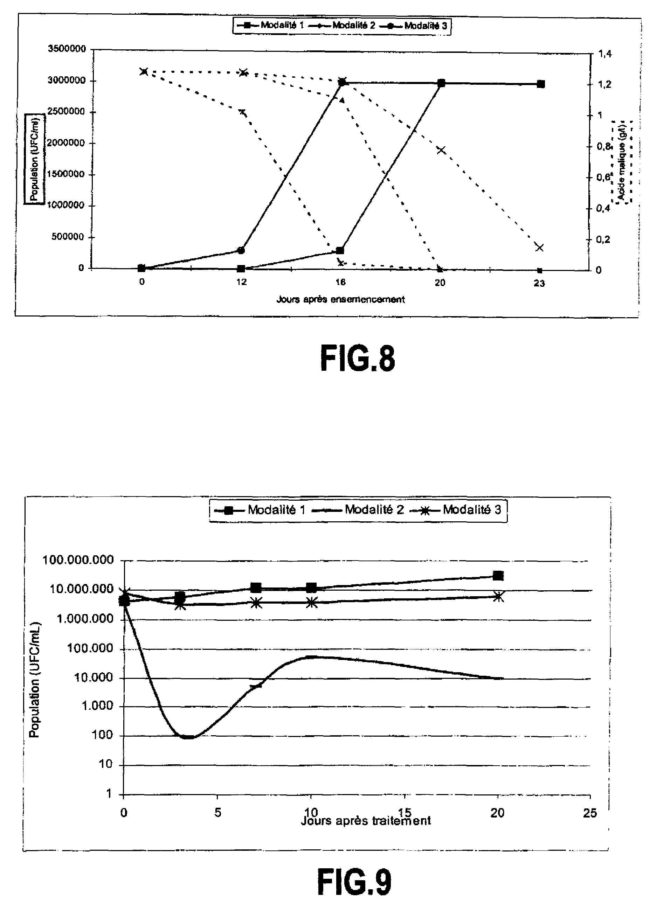

**Figure 8****- -** Evolution de la population en *Oenococcus oeni* et suivi de la dégradation en acide malique dans le vin avant et après le traitement au chitosane
**Légende** -
**Modalité 1 :** Vin témoin en fermentation malolactique en mode spontanée - pas de traitement au chitosane
**Modalité 2 :** Vin en fermentation malolactique en mode spontanée - traitement au chitosane 4g/hl
**Modalité 3 :** Vin en fermentation malolactique par inoculation - traitement au chitosane 4g/hl 8 jours avant l'inoculation

Un traitement au chitosane réalisé 8 jours avant l'inoculation en bactéries lactiques n'a pas d'impact sur la réalisation de la fermentation malolactique (modalité 3).

Dans le cas d'une fermentation malolactique spontanée (modalité 2), le traitement par le chitosane a pour effet de rallonger la phase de latence de la fermentation malolactique de 3 jours.

En conclusion, le chitosane n'a pas d'impact sur le déroulement de la fermentation malolactique (soit en mode spontanée, soit en inoculation). Toutefois, il est recommandé d'attendre 8 jours après la fin du traitement avant de réaliser une inoculation en bactéries lactiques.

### EXEMPLE 10- Traitement d'un vin rouge en cours de fermentation alcoolique contaminé en Brettanomyces bruxellensisà l'aide de chitosane à la dose de 4 g/hl, (cuve de 350hl)

Un vin rouge en cours de fermentation alcoolique languissante (Merlot) de la cave C présentant un niveau de population initiale en *Brettanomyces bruxellensis* <10UFC/ml a été traité par ajout de chitosane, à la dose de 4 g/hl. Le vin témoin ne subit aucun traitement.

**Tableau 4- Caractéristiques analytiques du vin**

| | Sucre (g/l) | TAV (%vol) | Ac.T (g/lH₂SO₄) | SO₂actif (mg/l) | pH | Acide malique (g/l) | Acide lactique (g/l) |
|---|---|---|---|---|---|---|---|
| Avant traitement | 12.4 | 14.1 | 4.3 | 0.24 | 3.53 | 1.03 | <0.4 |
| Après traitement | 9.9 | 14.26 | 4.39 | 0.19 | 3.55 | 1.03 | <0.4 |

On note que le chitosane ne modifie pas les paramètres d'analyses classiques après le traitement.

**Tableau 5- Dénombrement du niveau initial de contamination en Brettanomyces bruxellensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | <10 |
| Niveau de contamination du vin contrôle après 7 jours (T7) | 7 |
| Niveau de contamination du vin traité à l'aide de chitosane après 7 jours (TR7) | 1,05 |

Dans cet exemple, le taux de contamination initiale (T0) est faible (< 10UFC/ml). Toutefois, après 7 jours la population en *Brettanomyces bruxellensis* s'est développée dans l'échantillon non traité (T7).

Par contre, le traitement au chitosane a permis de réduire la population en *Brettanomyces bruxellensis* de l'échantillon traité (TR7).

Cet exemple démontre que le chitosane est efficace pour éliminer les *Brettanomyces bruxellensis* sur un vin en cours de fermentation alcoolique.

### EXEMPLE 11- Traitement d'un vin rouge en cours de fermentation malolactique languissante contaminé en Brettanomyces bruxellensisà l'aide de chitosane à la dose de 4 g/hl,(cuve de 200hl)

Un vin rouge en cours de fermentation malolactique (Merlot) de la cave SG présentant un niveau de population initiale en *Brettanomyces bruxellensis* <10UFC/ml a été traité par ajout de chitosane, à la dose de 4 g/hl. Le vin contrôle (T7) ne subit aucun traitement.

**Tableau 6- Caractéristiques analytiques du vin**

| | Sucre (g/l) | TAV (%vol) | Ac.T (g/l H₂SO₄) | SO₂actif (mg/l) | pH | Acide malique (g/l) | Acide lactique (g/l) |
|---|---|---|---|---|---|---|---|
| Avant traitement | 9.4 | 13.85 | 4 | 0.39 | 3.65 | 1.33 | 0.6 |
| Après traitement | 8.3 | 13.86 | 3.98 | 0.26 | 3.64 | 1.2 | 0.55 |

On note que le chitosane ne modifie pas les paramètres d'analyses classiques après le traitement.

**Tableau 7- Dénombrement du niveau initial de contamination en Brettanomyces bruxellensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | <10 |
| Niveau de contamination du vin contrôle après 7 jours (T7) | 10 |
| Niveau de contamination du vin traité à l'aide de chitosane après 7 jours (TR7) | <10 |

Dans cet exemple, le taux de contamination initiale (T0) est faible (< 10UFC/ml). Toutefois, après 7 jours la population en *Brettanomyces bruxellensis* s'est développée dans l'échantillon non traité (T7).

Par contre, le traitement au chitosane a permis de maintenir la population en *Brettanomyces bruxellensis* sous le seuil de détection (≤10UFC/ml).

Cet exemple démontre que le chitosane est efficace pour éliminer les *Brettanomyces bruxellensis* sur un vin en cours de fermentation malolactique languissante.

### EXEMPLE 12- Traitement d'un vin rouge en fin de fermentation malolactique présentant une contamination faible en Brettanomyces bruxellensis à l'aide de chitosane à la dose de 4 g/hl,(cuve de 130hl)

Un vin rouge en fin de fermentation malolactique (Assemblage Syrah Carignan Grenache) de la cave MT présentant un niveau de population initiale en *Brettanomyces bruxellensis* de ≤10UFC/ml a été traité par ajout de chitosane, à la dose de 4 g/hl. Le vin contrôle (T7) ne subit aucun traitement.

**Tableau 8- Caractéristiques analytiques du vin**

| | Sucre (g/l) | TAV (%vol) | Ac.T (g/lH₂SO₄) | SO₂actif (mg/l) | pH | Acide malique (g/l) | Acide lactique (g/l) |
|---|---|---|---|---|---|---|---|
| Avant traitement | 2.95 | 15.29 | 3.27 | 0.25 | 3.65 | <0.3 | 0.85 |
| Après traitement | 2.75 | 15.01 | 3.24 | 0.61 | 3.64 | <0.3 | 1 |

On note que le chitosane ne modifie pas les paramètres d'analyses classiques après le traitement.

**Tableau 9- Dénombrement du niveau initial de contamination en Brettanomyces bruxellensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | <10 |
| Niveau de contamination du vin contrôle après 7 jours (T7) | 80 |
| Niveau de contamination du vin traité à l'aide de chitosane après 7 jours (TR7) | 0,04 |

Bien que le taux de contamination initiale soit faible (≤10 UFC/ml), celui-ci s'est développé après sept jours dans l'échantillon contrôle. Par contre, le développement des *Brettanomyces bruxellensis* a pu être efficacement stoppé dans le vin traité à l'aide de chitosane.

Cet exemple démontre que le chitosane est efficace pour éliminer les *Brettanomyces bruxellensis* sur un vin en fin de fermentation malolactique dont la population initiale est faible.

### EXEMPLE 13- Traitement d'un vin rouge en fin de fermentation malolactique présentant une contamination modéré en Brettanomyces bruxellensisà l'aide de chitosane à la dose de 4 g/hl,(cuve de 45hl)

Un vin rouge en fin de fermentation malolactique (Assemblage Syrah Mourvèdre) de la cave MP présentant un niveau de population initiale en *Brettanomyces bruxellensis* de 30UFC/ml a été traité par ajout de chitosane, à la dose de 4 g/hl. Le vin contrôle (T7) ne subit aucun traitement.

**Tableau 10- Caractéristiques analytiques du vin**

| | Sucre (g/l) | TAV (%vol) | Ac.T (g/l H₂SO₄) | SO₂actif (mg/l) | pH | Acide malique (g/l) | Acide lactique (g/l) |
|---|---|---|---|---|---|---|---|
| Avant traitement | 1.8 | 14.39 | 2.97 | 0.15 | 3.75 | <0.3 | 1.4 |
| Après traitement | 1.85 | 14.29 | 3.02 | 0.19 | 3.76 | <0.3 | 1.4 |

On note que le chitosane ne modifie pas les paramètres d'analyses classiques après le traitement.

**Tableau 11- Dénombrement du niveau initial de contamination en Brettanomyces bruxellensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | 30 |
| Niveau de contamination du vin contrôle après 7 jours (T7) | >3000 |
| Niveau de contamination du vin traité à l'aide de chitosane après 7 jours (TR7) | <10 |

Malgré un taux de contamination initiale modéré (30UFC/ml), celle-ci a atteint après 7 jours un niveau très élevée (>3000 UFC/ml) dans l'échantillon non traité au chitosane. Par contre l'échantillon traité au chitosane présent une population en *Brettanomyces bruxellensis* ≤10UFC/ml.

Cet exemple démontre que le chitosane est efficace pour éliminer les *Brettanomyces bruxellensis* sur un vin en fin de fermentation malolactique dont la population initiale est modérée.

### EXEMPLE 14- Traitement d'un vin rouge en fin de fermentation malolactique présentant une forte contamination en Brettanomyces bruxellensisà l'aide de chitosane à la dose de 4 g/hl, (cuve de 500hl)

Un vin rouge en fin de fermentation malolactique (Syrah) de la cave R présentant un niveau de population initiale en *Brettanomyces bruxellensis* de 3400UFC/ml a été traité par ajout de chitosane, à la dose de 4 g/hl. Le vin contrôle (T7) ne subit aucun traitement.

**Tableau 12- Caractéristiques analytiques du vin**

| | Sucre (g/l) | TAV (%vol) | Ac.T (g/l H₂SO₄) | SO₂actif (mg/l) | pH | Acide malique (g/l) | Acide lactique (g/l) |
|---|---|---|---|---|---|---|---|
| Avant traitement | 1.9 | 12.63 | 3.3 | 0.86 | 3.72 | <0.3 | 1.4 |
| Après traitement | 1.8 | 12.6 | 3.29 | 0.79 | 3.73 | <0.3 | 1.4 |

On note que le chitosane ne modifie pas les paramètres d'analyses classiques après le traitement.

**Tableau 13- Dénombrement du niveau initial de contamination en Brettanomyces bruxelensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | 3400 |
| Niveau de contamination du vin contrôle après 7 jours (T7) | >3000 |
| Niveau de contamination du vin traité à l'aide de chitosane après 7 jours (TR7) | 20 |

On constate que le traitement au chitosane permet d'éliminer efficacement les *Brettanomyces bruxellensis* présentent dans le vin. Sept jours après le traitement, l'échantillon traité à l'aide de chitosane présente une population en *Brettanomyces bruxellensis* très faible (<20UFC/ml) comparativement à l'échantillon contrôle qui a maintenu un niveau de contamination élevé.

Cet exemple démontre que le chitosane est efficace pour éliminer les *Brettanomyces bruxellensis* sur un vin en fin de fermentation malolactique dont la population initiale est élevée.

### EXEMPLE 15- Traitement d'un vin rouge en fin de fermentation malolactique présentant une forte contamination en Brettanomyces bruxellensis à l'aide de chitosane à la dose de 4 g/hl, (cuve de 80hl)

Un vin rouge en fin de fermentation malolactique (Assemblage Syrah, Grenache) de la cave G présentant un niveau de population initiale en *Brettanomyces bruxellensis* de 1800UFC/ml a été traité par ajout de chitosane, à la dose de 4 g/hl. Le vin contrôle (T7) ne subit aucun traitement.

**Tableau 14- Caractéristiques analytiques du vin**

| | Sucre (g/l) | TAV (%vol) | Ac.T (g/l H₂SO₄) | SO₂actif (mg/l) | pH | Acide malique (g/l) | Acide lactique (g/l) |
|---|---|---|---|---|---|---|---|
| Avant traitement | 1.9 | 13.66 | 3.25 | 0.16 | 3.68 | <0.3 | 1.15 |
| Après traitement | 2 | 13.73 | 3.26 | 0.16 | 3.67 | <0.3 | 1.15 |

On note que le chitosane ne modifie pas les paramètres d'analyses classiques après le traitement.

**Tableau 15- Dénombrement du niveau initial de contamination en Brettanomyces bruxellensis (T0), de son évolution sur le vin non traité (T7) et le vin traité à l'aide de chitosane (TR7).**

| | Levures de type *Brettanomyces* (UFC/ml) |
|---|---|
| Niveau initiaux de contamination des vins (T0) | 1800 |
| Niveau de contamination du vin contrôle après 7 jours (T7) | > 3000 |
| Niveau de contamination du vin traité à l'aide de chitosane après 7 jours (TR7) | < 10 |

Le traitement au chitosane permet de faire chuter significativement la population en *Brettanomyces bruxellensis.* L'échantillon traité à l'aide de chitosane (TR7) présente une population en *Brettanomyces bruxellensis* ≤10UFC/ml comparativement à l'échantillon non traité dans lequel les *Brettanomyces bruxellensis* ont continué à croitre > 3000UFC/ml.

Cet exemple démontre que le chitosane est efficace pour éliminer les *Brettanomyces bruxellensis* sur un vin en fin de fermentation malolactique dont la population initiale est élevée.

### EXEMPLE 16- Traitement d'un cidre contaminé en Brettanomyces anomalus à l'aide de chitosane à la dose de 4 g/hl (essai laboratoire)

Un cidre commercial présentant un niveau de population initiale en *Brettanomyces anomalus* de 10⁷ cellules/ml a été traité par ajout de chitosane à la dose de 4 g/hl ou par ajout de DMDC à la dose de 20g/hl. Le vin témoin ne subit aucun traitement.

**Figure 9****-** Suivi de la population en *Brettanomyces anomalus* dans le cidre après le traitement
**Légende** -
**Modalité 1** : Témoin ;
**Modalité 2** : Traitement à l'aide de chitosane 4g/hl
**Modalité 3** : Traitement à l'aide de DMDC 20g/hl ;

On constate que seul le traitement à l'aide de chitosane (modalité 2) permet de réduire significativement le niveau de la population en *Brettanomyces anomalus* de 10⁷ UFC/ml à 100 UFC/ml en moins de 5 jours toutefois après 10 jours la population Brettanomyces anomalus a sensiblement augmentée pour atteindre de 5 10⁴UFC/ml.

Le traitement à l'aide de DMDC (modalité 3) n'a aucun effet sur les *Brettanomyces anomalus.*

### EXEMPLE 17- Traitement d'un jus de fruit contaminé en Brettanomyces bruxellensisà l'aide de chitosane à différentes doses (essai laboratoire)

Un jus de fruit commercial (joker) présentant une contamination en *Brettanomyces bruxellensis* a été traité par ajout de chitosane (DA≈4,2mol%) à différentes doses. Le jus de fruit témoin ne subit aucun traitement.

Après 7 jours de traitement, le chitosane permet une élimination totale des *Brettanomyces bruxellensis* présentes dans le jus de fruit.

### EXEMPLE 18- Traitement d'une matrice de moût pour la production d'alcool industriel contaminé en Brettanomyces bruxellensisà l'aide de chitosane à différentes doses (essai laboratoire)

Une matrice de moût pour la production d'alcool industriel présentant une contamination en *Brettanomyces bruxellensis* a été traitée par ajout de chitosane (DA≈4,2mol%) à différentes doses. La matrice témoin ne subit aucun traitement.

Après 7 jours de traitement, le chitosane permet une élimination totale des *Brettanomyces bruxellensis* présentes dans la matrice de moût industriel.

### EXEMPLE 19 - Traitement d'un vin rouge contaminé en Brettanomyces bruxellensisà l'aide de chitosane à la dose de 4 g/hl (essai laboratoire)

Un vin fini de Bordeaux millésime 2010 présentant un niveau de population initiale en *Brettanomyces bruxellensis* de 1.2 10³ cellules/ml a été traité par ajout de chitosane à la dose de 4 g/hl.

**Tableau 16: Suivi de l'efficacité du traitement chitosane à la dose de 4g/hl par dénombrement par PCR quantitative et dénombrement sur milieu sélectif**

| | | Dénombrement par PCR quantitative (UFC/ml) | Dénombrement sur milieu sélectif (UFC/ml) |
|---|---|---|---|
| Bordeaux 2010 | Avant traitement | 12 000 | / |
| | 10 jours après traitement | 5000 | <10 |
| | 20 jours après traitement | 3300 | < 10 |
| | 30 jours après traitement | < 10 | < 10 |

Les résultats présentés dans le tableau 16 montrent une différence en fonction de la méthode d'analyse : alors que par mise en culture sur milieu gélosé, la population viable est estimée à moins de 10 unités formant colonies dès le premier contrôle (10 jours post traitement), il faut attendre 30 jours post traitement avec l'analyse par RT-PCR pour obtenir ce résultat.

Ceci illustrerait que le chitosane interagit vraisemblablement avec la paroi membranaire des cellules de *Brettanomyces* entrainant une déstructuration de cette dernière; ce mécanisme induirait une réponse des cellules comparable à un état sub-létal, précédant leur mort. Dans cet état sub-létal, les cellules seraient identifiées comme viables par la PCR quantitative pendant plusieurs jours avant leur mort totale alors que l'analyse sur milieu gélosé les identifierait de suite comme non cultivables.

### EXEMPLE 20 - Traitement d'un vin rouge contaminé en Brettanomyces bruxellensisà l'aide de chitosane à la dose de 8 g/hl (essai laboratoire)

Un vin fini de la Vallée du Rhône millésime 2009 présentant un niveau de population initiale en *Brettanomyces bruxellensis* supérieure à 10³ cellules/l a été traité par ajout de chitosane à la dose de 8 g/hl au départ d'une suspension à 20%.

Pour cet essai, le chitosane est pesé et mélangé dans 5ml d'eau distillée (suspension mère à 20%). 100µl de cette suspension mère sont prélevés puis ajouter au vin à traiter à raison de 8g/hl (en poids de chitosane par rapport au volume de liquide total) pour un volume de 250mL.

**Tableau 17- Caractéristiques analytiques du vin**

| Sucre (g/l) | TAV (%vol) | Ac.T (g/l H₂SO₄) | SO₂ actif(mg/l) | pH |
|---|---|---|---|---|
| 1,33 | 13,85 | 2,77 | 0,39 | 3,75 |

**Tableau 18- Dénombrement sur milieu culture des Brettanomyces bruxellensis 10 jours après traitement**

| | Levures de type *Brettanomyces* (UFC/l) |
|---|---|
| Niveau initial de contamination du vin (T0) | > 10 000 |
| Niveau de contamination du vin traité à l'aide de chitosane après 10 jours (TR10) | < 1 |

Après 10 jours de traitement, le chitosane à la dose de 8g/hl permet une élimination totale des *Brettanomyces bruxellensis* présentes dans le vin.

### EXEMPLE 21 - Traitement d'un vin rouge contaminé en Brettanomyces bruxellensis à l'aide de chitosane à la dose de 4 g/hl (essai laboratoire)

Un vin fini de la Vallée du Rhône millésime 2009 présentant un niveau de population initiale en *Brettanomyces bruxellensis* supérieure à 270 cellules/ml a été traité par ajout de chitosane à la dose de 4 g/hl au départ d'une suspension à 20%.

Pour cet essai, le chitosane est pesé et mélangé dans 5ml d'eau distillée (suspension mère à 20%). 50µl de cette suspension mère sont prélevés puis ajouter au vin à traiter.

**Tableau 19- Caractéristiques analytiques du vin**

| Sucre (g/l) | TAV (%vol) | Ac.T (g/l H₂SO₄) | SO₂ actif(mg/l) | pH |
|---|---|---|---|---|
| 1,35 | 13,74 | 2,70 | 0,26 | 3,86 |

**Tableau 20- Dénombrement par q-PCR des Brettanomyces bruxellensis 10 jours après traitement**

| | Levures de type *Brettanomyces* (UFC/ml) |
|---|---|
| Niveau initial de contamination du vin (T0) | 270 |
| Niveau de contamination du vin traité à l'aide de chitosane après 10 jours (TR10) | Non détecté |

Après 10 jours de traitement, le chitosane à la dose de 4g/hl permet une élimination totale des *Brettanomyces bruxellensis* présentes dans le vin.

### EXEMPLE 22- Traitement d'un vin rouge contaminé en Brettanomyces bruxellensis à l'aide de chitosane à la dose de 4 g/hl (essai laboratoire)

Un vin fini de Bordeaux millésime 2003 présentant un niveau de population initiale en *Brettanomyces bruxellensis* de 10⁵ cellules/ml a été traité par ajout de chitosane à la dose de 4 g/hl. Le vin témoin ne subit aucun traitement. L'essai a été dupliqué.

**Tableau 21 : Dénombrement sur milieu de culture sélectif des populations de levures cultivables dans les vins soutirés et réduction logarithmique de la population initiale 10 jours après le traitement**

| | **Dénombrement des levures cultivables (UFC/ml)** | **Réduction logarithmique** |
|---|---|---|
| Témoin (1ère répétition) | 6.8 10⁵ | / |
| Témoin (2de répétition) | 5.7 10⁵ | / |
| Chitosane 4g/hl (1 ère répétition) | Non détecté | Supérieure 5 |
| Chitosane 4g/hl (2de répétition) | Non détecté | Supérieure 5 |

Les résultats mettant en oeuvre le témoin et présentés dans le Tableau 21 démontrent que des populations élevées de *Brettanomyces bruxellensis* persistent. En revanche, dans les résultats des modalités traitées au chitosane, on note qu'aucune population de levure cultivable n'est détectée. La réduction logarithmique est calculée en prenant comme référentiel la population cultivable présente dans le vin juste avant le traitement (2.1 10⁵ UFC/ml). Cette réduction logarithmique est toujours supérieure à 5.

**Tableau 22 : Dénombrement par microscopie à épifluorescence des populations de levures viables et non viables réalisé sur les lies 10 jours après le traitement et le pourcentage de cellules vivantes parmi les lies représentant le rapport des cellules vivantes sur la totalité des cellules dénombrées dans les lies**

| | **Levures vivantes (cellules/ml)** | **Levures mortes (cellules/ml)** | **% cellules vivantes parmi les lies** |
|---|---|---|---|
| Témoin (1 ère répétition) | 4.8 10⁶ | Aucunes levures mortes | / |
| Témoin (2de répétition) | 8.9 10⁶ | Aucunes levures mortes | / |
| Chitosane 4g/hl (1ère répétition) | 6.1 10⁴ | 2.4 10⁶ | 2.5 |
| Chitosane 4g/hl (2de répétition) | 6.1 10⁴ | 3.2 10⁶ | 1.9 |

L'analyse des lies par microscopie à épifluorescence 10 jours après le traitement (Tableau 22) démontre que dans les témoins non traités aucune cellule morte n'est comptabilisée.

A contrario dans les modalités traitées au chitosane à la dose de 4g/hl, une forte proportion de levures détectées est morte et le pourcentage de levures vivantes est très faible.

### EXEMPLE 23- Traitement d'un vin rouge contaminé en Brettanomyces bruxellensis de différentes granulométries et degré acétylation (DA) à la dose de 4 g/hl (essai laboratoire)

Un vin fini de Bordeaux millésime 2003 présentant un niveau de population initiale en *Brettanomyces bruxellensis* de 10⁵ cellules/ml a été traité par ajout de chitosane de différentes granulométries et degré acétylation : <50µm, DA = 15 (% mol) (Formule A) et < 90µm, DA = 15(% mol) (Formule B) à la dose de 4g/hl. Le vin témoin ne subit aucun traitement. L'essai a été dupliqué.

**Tableau 23 : Dénombrement sur milieu de culture sélectif des populations de levures cultivables dans les vins soutirés et réduction logarithmique de la population initiale 10 jours après le traitement**

| | **Dénombrement des levures cultivables (UFC/ml)** | **Réduction logarithmique** |
|---|---|---|
| Témoin (1 ère répétition) | 6.8 10⁵ | / |
| Témoin (2de répétition) | 5.7 10⁵ | / |
| **Formule A** 4g/hl (1ère répétition) | Non détecté | Supérieure 5 |
| **Formule A** 4g/hl (2de répétition) | Non détecté | Supérieure 5 |
| **Formule B** 4g/hl (1ère répétition) | Non détecté | Supérieure 5 |
| **Formule B** 4g/hl (1ère répétition) | Non détecté | Supérieure 5 |

Les résultats des témoins présentés dans le Tableau 23 démontrent que des populations élevées de *Brettanomyces bruxellensis* persistent. En revanche, des résultats des modalités traitées au chitosane, on note qu'aucune population de levure cultivable n'est détectée. La réduction logarithmique est calculée en prenant comme référentiel la population cultivable présente dans le vin juste avant le traitement (2.1 10⁵ UFC/ml). Cette réduction logarithmique est toujours supérieure à 5.

**Tableau 24 : Dénombrement par microscopie à épifluorescence des populations de levures viables et non viables réalisé sur les lies 10 jours après le traitement et le pourcentage de cellules vivantes parmi les lies représentant le rapport des cellules vivantes sur la totalité des cellules dénombrées dans les lies**

| | **Levures vivantes (cellules/ml)** | **Levures mortes (cellules/ml)** | **% cellules vivantes parmi les lies** |
|---|---|---|---|
| Témoin (1ère répétition) | 4.8 10⁶ | Aucunes levures mortes | / |
| Témoin (2de répétition) | 8.9 10⁶ | Aucunes levures mortes | / |
| **Formule A** 4g/hl (1 ère répétition) | 1.5 10⁴ | 9.1 10⁵ | 1.6 |
| **Formule A** 4g/hl (2de répétition) | 1.5 10⁴ | 1.6 10⁶ | 0.9 |
| **Formule B** 4g/hl (1 ère répétition) | 3.1 10⁴ | 7.0 10⁵ | 4.2 |
| **Formule B** 4g/hl (1 ère répétition) | 6.3 10⁴ | 1.5 10⁶ | 4.0 |

L'analyse des lies par microscopie à épifluorescence 10 jours après le traitement (tableau 24) démontre que dans les témoins non traités aucune cellule morte n'est comptabilisée.

A contrario dans les modalités traitées au chitosane à la dose de 4g/hl, une forte proportion de levures détectées est morte. Le pourcentage de levures vivantes est faible quelque soit la formule. Cependant on note que la formule B (granulométrie < 90µm) présente un pourcentage de levures vivantes supérieure à celui de la formule A (granulométrie <50µm).

### EXEMPLE 24- Solubilité du chitosane - Pureté et résidus solubles- Dosage par la méthode du Codex oenologique

Le chitosane selon l'invention est pratiquement totalement insoluble.

Le pourcentage de matières insolubles doit être égale ou supérieure à 95 %. Elle est déterminée de la façon suivante (tirée de la monographie OIV du chitosane ajoutée Codex OEnologique international) :

Placer en solution 5 g de chitosane dans 100 ml d'eau bi-distillée et agiter 2 minutes. Filtrer après refroidissement sur filtre serré ou sur membrane. Evaporer le filtrat et sécher à 100-105 °C. La teneur en matières solubles ne devrait pas être supérieure à 5 %.

Le % de matières insolubles mesuré sur 10 lots de chitosane est reporté dans le tableau 25 ci-dessous.

| Numéro de lot du chitosane | % d'insolubles |
|---|---|
| 1 | 96,79% |
| 2 | 94,50% |
| 3 | 97,76% |
| 4 | 96,42% |
| 5 | 98,54% |
| 6 | 95,06% |
| 7 | 98,62% |
| 8 | 95,61% |
| 9 | 97,19% |
| 10 | 95,42% |

### EXEMPLE 25 - Calcul du degré de cristallinité.

Le degré de cristallinité d'un chitosane selon l'invention, complètement hydraté est mesuré par diffraction aux rayons X. La poudre de chitosane est hydratée en la plaçant dans excès d'eau déionisée (10 fois son poids) pendant 24h. Un diffractomètre Siemens D5000 (rayonnement Cu Kalpha, 40 kV, 45 mA, fente variable de divergence V20, filtre Ni + fentes de 0.6 mm et 0.2 mm devant le détecteur) est utilisé. Les données sont collectées en utilisant un mode step scan de 2 à 60 degrés 2theta à 2.5 sec/step, et un step size de 0.04°.

Le degré de cristallinité est calculé par le rapport entre l'aire des pics cristallins et la somme des aires des pics cristallins et de la région amorphe enter 7 et 49 degrés 2theta. Le résultat reporté est la moyenne de 2 mesures sur un lot de chitosane.

**Tableau 26**

| Degré de cristallinité | |
|---|---|
| Avant hydratation | Après hydratation |
| 26% | 6% |

### EXEMPLE 26- dosage du chitosane résiduel dans un vin traité

Un vin traité par du chitosane a été filtré sur Buchner à l'aide d'un filtre 1µm type Pall. Le résidu obtenu après filtration a ensuite été lavé à l'eau (Ph. Eur.). Le résidu présent sur le filtre est ensuite récupéré, placé dans un Falcon et lavé à l'eau (Ph. Eur.) par centrifugation jusqu'à obtenir une conductimètre du surnageant inférieur à 100µS. Le résidu est congelé, lyophilisé et récupéré (100mg). Il est mis en suspension dans 10ml d'eau + 50µl acide lactique. L'échantillon est dilué dans l'acide acétique 1% puis injectée en HPLC-ELSD (chromatographie liquide à haute performance couplée à un détecteur universel ELSD (Evaporative Light Scattering Detector) et dosé par rapport à un étalonnage avec un standard de chitosane. Le résultats de l'analyse est le suivant :

Teneur en chitosane dans l'échantillon : inférieur à la limite de détection (10mg/l).

## Revendications

1. Utilisation de chitosane issu d'un champignon du type *Ascomycète,* et/ou d'un champignon *Basidiomycète,* dont la granulométrie est comprise entre 0.1 et 200 micromètres, ou d'une composition en comprenant, en tant qu'antimicrobien pour traiter un liquide alimentaire d'origine végétale préparé par fermentation.

2. Utilisation, selon la revendication 1, **caractérisé en ce que** le chitosane est utilisé en tant qu'antifongique pour lutter contre les levures indésirables dans le liquide alimentaire.

3. Utilisation, selon la revendication 1 ou 2, **caractérisée en ce que** le chitosane est issu *d'Aspergillus niger,* de *Lentinula edodes* (shiitake), ou d'*Agaricus bisporus.*

4. Utilisation, selon l'une quelconque des revendications 1 à 3, **caractérisée en ce que** le liquide alimentaire d'origine végétale préparé par fermentation est un vin, une bière, un cidre, un vin effervescent, ou un jus de fruit.

5. Utilisation, selon l'une quelconque des revendications 1 à 4, **caractérisée en ce que** le chitosane est utilisé à une concentration de 1 à 10 g/hL de liquide alimentaire à traiter.

6. Utilisation, selon l'une quelconque des revendications 1 à 4, **caractérisée en ce que** le chitosane est utilisé à une concentration comprise entre 2 et 5g/hL de liquide alimentaire à traiter.

7. Méthode de traitement curatif et/ou préventif d'un liquide alimentaire d'origine végétale préparé par fermentation, dans laquelle des levures indésirables sont à éliminer, dont la présence est à prévenir, et/ou dont la population est à limiter, ladite méthode comprenant la mise en présence du liquide alimentaire avec un chitosane issu d'un champignon du type *Ascomycète,* et/ou d'un champignon *Basidiomycète,* dont la granulométrie est comprise entre 0.1 et 200 micromètres, ou d'une composition en comprenant.

8. Méthode, selon la revendication 7, **caractérisée en ce que** le chitosane est issu *d'Aspergillus niger,* de *Lentinula edodes* (shiitake), ou d'*Agaricus bisporus.*

9. Méthode, selon la revendication 7 ou 8, **caractérisée en ce que** le liquide alimentaire d'origine végétale préparé par fermentation est un vin, une bière, un cidre, un vin effervescent, ou un jus de fruit.

10. Méthode, selon l'une quelconque des revendications 7 à 9, **caractérisée en ce que** les levures indésirables sont des levures du genre Brettanomyces et/ou Dekkera.

11. Méthode, selon l'une quelconque des revendications 7 à 10, **caractérisée en ce que** les levures indésirables sont *B. anomalus et*/*ou B. bruxellensis.*

12. Méthode, selon l'une quelconque des revendications 7 à 11, **caractérisée en ce que** le chitosane est présent à une concentration de 1 à 10 g/hL de liquide alimentaire à traiter.

13. Méthode, selon l'une quelconque des revendications 7 à 12, **caractérisée en ce que** le chitosane est présent à une concentration comprise entre 2 et 5g/hL de liquide alimentaire à traiter.

14. Méthode, selon l'une quelconque des revendications 7 à 13, **caractérisée en ce que** le liquide alimentaire est mise en présence de chitosane pendant au moins 3 jours.

15. Méthode, selon l'une quelconque des revendications 7 à 14, **caractérisée en ce que** le chitosane et/ou la composition comprenant le chitosane, est séparé(e) du liquide alimentaire pour conserver le liquide alimentaire.
